(19) Europäisches Patentamt
European Patent Office
Office européen des brevets

(11) **EP 3 690 443 A1**

(12) **EUROPEAN PATENT APPLICATION**

(43) Date of publication:
05.08.2020 Bulletin 2020/32

(21) Application number: 20164877.1

(22) Date of filing: 09.12.2015

(51) Int Cl.:
*G01N 33/543* (2006.01)   *G01N 33/531* (2006.01)
*G01N 33/533* (2006.01)   *G01N 21/64* (2006.01)
*G01N 33/574* (2006.01)   *G01N 33/58* (2006.01)
*G01N 33/536* (2006.01)   *G01N 33/53* (2006.01)
*G01N 33/48* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 12.12.2014 JP 2014251979

(62) Document number(s) of the earlier application(s) in accordance with Art. 76 EPC:
**15868287.2 / 3 232 195**

(71) Applicant: **Konica Minolta, Inc.**
**Tokyo 100-7015 (JP)**

(72) Inventors:
• **GOUDA, Hideki**
  **Tokyo 100-7015 (JP)**
• **TAKANASHI, Kensaku**
  **Tokyo 100-7015 (JP)**

(74) Representative: **Henkel & Partner mbB**
**Patentanwaltskanzlei, Rechtsanwaltskanzlei**
**Maximiliansplatz 21**
**80333 München (DE)**

Remarks:
This application was filed on 23.03.2020 as a divisional application to the application mentioned under INID code 62.

(54) **DILUENT FOR FLUORESCENT NANO PARTICLES, KIT FOR IMMUNOFLUORESCENT STAINING WHICH UTILIZES SAME, SOLUTION FOR IMMUNOFLUORESCENT STAINING, IMMUNOFLUORESCENT STAINING METHOD, AND GENE STAINING METHOD**

(57)   [Problem]
Provided is a means for detecting and quantifying a biological substance of interest with an improved accuracy by inhibiting non-specific adsorption of fluorescent nanoparticles and thereby reducing the background noise in immunostaining with fluorescent nanoparticles.
[Means for Solution]
Immunostaining is carried out upon diluting fluorescent nanoparticles with a fluorescent nanoparticle diluent which contains 1 to 5% (W/W) of a protein having a molecular weight of 40, 000 or higher (e.g., BSA) and 1 to 3% (W/W) of a protein having a molecular weight of less than 40, 000, wherein said protein having a molecular weight of less than 40,000 is casein. It is preferred that the $\kappa$-casein content in the casein is 10%(W/W) or less and the ratio of $\alpha$-casein and $\beta$-casein ($\alpha$-casein:$\beta$-casein) contained in the casein is 40:60 to 60:40 (taking the total amount of $\alpha$-casein and $\beta$-casein as 100).

EP 3 690 443 A1

**Description**

TECHNICAL FIELD

The present invention relates to: a fluorescent nanoparticle diluent used in an immunofluorescent staining method (or a gene detection method); an immunofluorescent staining kit and an immunofluorescent staining solution, which comprise the same; and an immunofluorescent staining method and a gene staining method.

BACKGROUND ART

With the recent expansion of molecular target drug therapy mainly based on antibody drugs, there is an increasing need for an accurate diagnostic method for more efficient use of molecular target drugs. Specifically, it is demanded to efficiently determine whether or not a molecular target drug is applicable to each patient by quantitatively evaluating the expression of a target biological substance. For the determination of the effectiveness of certain drug administration, for example, immunohistochemistry [IHC] methods which analyze the expression of a protein or the like as a target biological substance and FISH [fluorescence *in situ* hybridization] methods which analyze the amplification of a target biological substance gene or the like have been widely employed in the clinical settings.

At present, in many cases, a tissue collected from an affected part is subjected to treatments such as dehydration and blocking with paraffin for fixation and subsequently cut into a section of 2 to 8 $\mu$m in thickness, after which paraffin is removed from the section (hereinafter, also referred to as "tissue section") and the section is subjected to staining of a target biological substance, followed by observation thereof under a microscope. In the thus obtained micrograph, a diagnosis is made on the basis of the morphological information and staining information, such as changes in the size and shape of cell nuclei and changes in tissue pattern. For such pathological diagnosis, the development of image digitalization technology has enable to propose automated pathological diagnosis support equipments that display information required for pathological diagnosis by a pathologist through extraction and measurement of a pathological image that is input as a digital color image using a microscope, a digital camera and the like.

Conventionally, as tissue staining methods, hematoxylin-eosin [HE] staining using a dye and DAB staining using an enzyme have been widely employed; however, since the staining concentration in these methods is greatly affected by environmental conditions such as temperature and time, it is considered difficult to achieve an accurate quantitative measurement.

Meanwhile, Patent Document 1 discloses an immunostaining method which uses fluorescent nanoparticles as a labeling reagent in place of a dye. By performing immunostaining with fluorescent nanoparticles, evaluation can be performed with such a high accuracy and quantitative performance that could not be achieved by a conventional enzyme method; however, when such a high-brightness phosphor is used, since even a single particle thereof is detectable, there is a problem that non-specific binding of the fluorescent nanoparticles is likely to generate background noise (this problem also similarly occurs in the detection of, for example, a disease-associated gene (e.g., HER2 gene)) .

In order to improve the accuracy of a quantitative immunostaining method (and a gene staining method) using fluorescent nanoparticles, the present inventors have devised ingenious ideas, such as an addition of a protein to the subject section in the blocking step as described in Patent Document 1. However, the present inventors have not particularly examined the composition of a fluorescent nanoparticle diluent. As described in Patent Document 2, for example, 1% BSA-containing PBS that is widely applied as an antibody diluent has been used for diluting fluorescent nanoparticles.

RELATED ART DOCUMENTS

PATENT DOCUMENTS

[Patent Document 1] JP 2013-57631 A
[Patent Document 2] JP 2006-53031 A

SUMMARY OF THE INVENTION

PROBLEMS TO BE SOLVED BY THE INVENTION

An object of the present invention is to solve the above-described problems by providing a means for performing detection and quantification of a biological substance of interest with an improved accuracy through inhibition of non-specific adsorption of fluorescent nanoparticles and reduction of background noise in immunostaining (or gene staining) with fluorescent nanoparticles.

TECHNICAL SOLUTION

As a result of intensively studying to solve the above-described problems, the present inventors came to focus on the composition of a fluorescent nanoparticle diluent employed in an immunofluorescent staining method (or a gene staining method) using fluorescent nanoparticles and, as a result of further studies, the present inventors discovered that non-specific adsorption of fluorescent nanoparticles can be inhibited by dispersing the fluorescent nanoparticles in a solution containing a high-molecular-weight protein (Mw ≥ 40,000: e.g., BSA) and a low-molecular-weight protein (Mw < 40,000: e.g., casein) each at a prescribed concentration, thereby completing the present invention.

In order to realize the above-described object, the fluorescent nanoparticle diluent which reflects one aspect of the present invention comprises 1 to 5%(W/W) of a protein having a molecular weight of 40,000 or higher and 1 to 3% (W/W) of a protein having a molecular weight of less than 40,000, which are used for performing immunostaining (or gene staining) with fluorescent nanoparticles.

ADVANTAGEOUS EFFECTS OF THE INVENTION

In immunofluorescent staining (or gene staining), by using the fluorescent nanoparticle diluent of the present invention to dilute fluorescent particles, the background noise observed at the time of detection can be reduced, so that the accuracy and the quantitative performance in the evaluation of a stained image can be improved.

MODE FOR CARRYING OUT THE INVENTION

Embodiments of the present invention will now be described; however, the present invention is not restricted thereto.

-Fluorescent Nanoparticle Diluent-

The fluorescent nanoparticle diluent of the present invention comprises, at least: 1 to 5%(W/W) of a high-molecular-weight (Mw ≥ 40,000) protein; and 1 to 3% (W/W) of a low-molecular-weight (Mw < 40,000) protein and, by dispersing fluorescent nanoparticles using this solution, an immunofluorescent staining solution (or gene staining solution) for performing immunostaining (or gene staining) can be prepared. The present invention will now be described in more detail.

<Diluent>

The fluorescent nanoparticle diluent of the present invention comprises, at least, 1 to 5%(W/W) of a high-molecular-weight (Mw ≥ 40,000) protein and/or 1 to 3%(W/W) of a low-molecular-weight (Mw < 40,000) protein.

The high-molecular-weight protein is preferably BSA (molecular weight: about 66,000). BSA exerts an effect of stabilizing proteins in particular. The upper limit of the molecular weight of the high-molecular-weight protein is not particularly restricted; however, it is usually 400,000. When the concentration of the high-molecular-weight protein is less than 1%, the number of bright sports to be observed is reduced and signals are weakened. Meanwhile, when the concentration of this protein is higher than 5%, bright spots are likely to aggregate with each other, making it difficult to accurately evaluate the signals. From the standpoint of such actions and effects, the concentration of the high-molecular-weight protein is more preferably 1.5 to 3%(W/W).

The low-molecular-weight protein is preferably casein (molecular weight: about 23,000). The use of casein particularly exerts an effect of blocking those parts to which a protein non-specifically adsorbs. The lower limit of the molecular weight of the low-molecular-weight protein is not particularly restricted; however, it is usually 1,000. When the concentration of the low-molecular-weight protein is lower than 1%, the background noise is increased and this leads to a reduction in the signal quantitative performance. Meanwhile, when the concentration of this protein is higher than 3%, the number of bright sports to be observed is reduced and signals are weakened. From the standpoint of such actions and effects, the concentration of the low-molecular-weight protein is more preferably 1.2 to 2.4%(W/W).

In natural caseins contained in cow's milk and the like, four types of caseins, which are $\alpha$-casein, $\beta$-casein, $\kappa$-casein and $\gamma$-casein, are contained at a ratio of 50%, 35%, 13% and 2%, respectively. These caseins may each be commercially available or can be fractionated by a variety of fractionation methods, and caseins of a desired composition can be prepared by mixing the above-described caseins.

By adjusting the ratio of $\alpha$-casein and $\beta$-casein that are contained in the fluorescent nanoparticle diluent of the

present invention ($\alpha$-casein:$\beta$-casein) to be 40:60 to 60:40 (taking the total amount of $\alpha$-casein and $\beta$-casein as 100) and/or adjusting the content of $\kappa$-casein in the caseins to be 10% or less, the effects of the background in observation are further reduced and evaluation can be made with superior quantitative performance. Caseins are known to form a variety of micellar structures, particularly with $\kappa$-casein at the center, and it is possible that a composition satisfying the above-described conditions contributes to the actions and effects of the present invention through such a mode of micellar structure formation.

From the above, it is preferred that, specifically, the fluorescent nanoparticle diluent of the present invention comprise 1 to 5%(W/W) of BSA, 1 to 3%(W/W) of caseins; and that the ratio of $\kappa$-casein contained in the caseins be 10% (W/W) or less (particularly, it is more preferred that the $\kappa$-casein ratio be 0.5 to 10%(W/W)). Further, it is particularly preferred that the ratio of $\alpha$-casein and $\beta$-casein ($\alpha$-casein:$\beta$-casein) be 40:60 to 60:40 (taking the total amount of $\alpha$-casein and $\beta$-casein as 100) .

For adjustment of the concentrations of the respective proteins as described above, a solvent (buffer) such as PBS or TBS is generally used. That is, these buffers can be used as a solvent of the fluorescent nanoparticle diluent of the present invention.

The fluorescent nanoparticle diluent may further contain a nonionic surfactant such as Tween 20 or Digitonin, and a chelating reagent such as EDTA.

By using this fluorescent nanoparticle diluent to dilute a fluorescent nanoparticle-containing immunostaining reagent for a biological substance of interest, the background noise is reduced, so that highly quantitative immunofluorescent staining (or gene staining) can be performed.

• Kit

In addition, in the present invention, a kit for performing immunostaining (or gene staining) with fluorescent nanoparticles is prescribed. This kit comprises, at least: a fluorescent nanoparticle-containing immunostaining reagent (or gene staining reagent) for a biological substance of interest; and the above-described fluorescent nanoparticle diluent. This kit may further comprise, as required, for example, other reagents, members and the like that are necessary for immunostaining the biological substance of interest, and an instruction manual for carrying out the immunofluorescent staining (or gene staining) according to the present invention.

• Staining Solution

Further, the present invention provides an immunofluorescent staining solution (or gene staining solution) obtained by diluting an immunostaining reagent for a biological substance of interest with the above-described fluorescent nanoparticle diluent. The dilution factor of the immunostaining reagent (or gene staining reagent) can be optimized in accordance with the affinity between the biological substance of interest and the immunostaining reagent.

<Biological Substance of Interest>

In the present invention, the biological substance of interest is a biological substance, particularly a protein (antigen) (or a gene) that is expressed in a tissue section, and refers to a subject of immunostaining performed with a fluorescent label for the purpose of quantification or detection mainly from the standpoint of pathological diagnosis.

The biological substance of interest is not particularly restricted and may be selected taking into consideration the use of the quantification method of the present invention, such as pathological diagnosis. Examples of a typical biological substance of interest include biological substances that are expressed on the cell membranes of various cancer tissues and can be utilized as biomarkers, such as growth factor receptors (e.g., EGFR (HER1) (Epidermal Growth Factor Receptor), HER2 (Human Epidermal Growth Factor Receptor), HER3, HER4, VEGFR (Vascular Endothelial Growth Factor Receptor), IGFR (Insulin-like Growth Factor Receptor), and HGFR (Hepatocyte Growth Factor Receptor)), and proteins serving as immune system receptors (e.g., PD-1 (Programmed cell death 1) and PD-L1 (Programmed cell death ligand 1)). Examples of EGFR/HER include EGFR/HER1 (also called "ErbB1") which is overexpressed in cancer tissues such as colon cancer, EGFR2/HER2 (also called "ErbB2" or "neu") which is overexpressed in cancer tissues such as breast cancer, EGFR3/HER3, and EGFR4/HER4. Examples of VEGFR include VEGFR-1 (also called "Flt-1") and VEGFR-2 (also called "Flt-2" or "KDR"), which show enhanced expression in vascular endothelial cells of cancer tissues such as liver cancer and esophageal cancer, and VEGFR-3 (also called "Flt-4") which shows enhanced expression in lymphatic endothelial cells. For example, HER2 is suitable as the biological substance of interest when the quantification method of the present invention is performed in pathological diagnosis relating to breast cancer.

In addition to the above, examples of the biological substance of interest also include HER2, TOP2A, HER3, EGFR, P53 and MET as genes relating to the cancer growth or the efficiency of a molecular target drug, as well as the followings as genes that are known as cancer-related genes. Examples of tyrosine kinase-related genes include ALK, FLT3, AXL, FLT4 (VEGFR3), DDR1, FMS (CSF1R), DDR2, EGFR (ERBB1), HER4 (ERBB4), EML4-ALK, IGF1R, EPHA1, INSR, EPHA2, IRR (INSRR), EPHA3, KIT, EPHA4, LTK, EPHA5, MER (MERTK), EPHA6, MET, EPHA7, MUSK, EPHA8, NPM1-ALK, EPHB1, PDGFR$\alpha$ (PDGFRA), EPHB2, PDGFR$\beta$(PDGFRB), EPHB3, RET, EPHB4, RON (MST1R), FGFR1, ROS (ROS1), FGFR2, TIE2 (TEK), FGFR3, TRKA (NTRK1), FGFR4, TRKB (NTRK2), FLT1 (VEGFR1), and TRKC (NTRK3). Further, examples of breast cancer-related genes include ATM, BRCA1, BRCA2, BRCA3, CCND1, E-Cadherin, ETV6, FGFR1, HRAS, KRAS, NRAS, NTRK3, p53, and PTEN. Examples of carcinoid tumor-related genes include BCL2, BRD4, CCND1, CDKN1A, CDKN2A, CTNNB1, HES1, MAP2, MEN1, NF1, NOTCH1, NUT, RAF, SDHD, and VEGFA. Examples of colon cancer-related genes include APC, MSH6, AXIN2, MYH, BMPR1A, p53, DCC, PMS2, KRAS2 (or Ki-ras), PTEN, MLH1, SMAD4, MSH2, STK11, and MSH6. Examples of lung cancer-related genes include ALK, PTEN, CCND1, RASSF1A, CDKN2A, RB1, EGFR, RET, EML4, ROS1, KRAS2, TP53, and MYC. Examples of liver cancer-related genes include Axin1, MALAT1, b-catenin, p16 INK4A, c-ERBB-2, p53, CTNNB1, RB1, Cyclin D1, SMAD2, EGFR, SMAD4, IGF2, TCF1, and KRAS. Examples of renal cancer-related genes include Alpha, PRCC, ASPSCR1, PSF, CLTC, TFE3, p54nrb/NONO, and TFEB. Examples of thyroid cancer-related genes include AKAP10, NTRK1, AKAP9, RET, BRAF, TFG, ELE1, TPM3, H4/D10S170, and TPR. Examples of ovarian cancer-related genes include AKT2, MDM2, BCL2, MYC, BRCA1, NCOA4, CDKN2A, p53, PIK3CA, GATA4, RB, HRAS, RET, KRAS, and RNASET2. Examples of prostate cancer-related genes include AR, KLK3, BRCA2, MYC, CDKN1B, NKX3.1, EZH2, p53, GSTP1, and PTEN. Examples of bone tumor-related genes include CDH11, COL12A1, CNBP, OMD, COL1A1, THRAP3, COL4A5, and USP6.

<Antibody>

As a primary antibody, an antibody (IgG) which specifically recognizes and binds to a protein, which is the above-described biological substance of interest, as an antigen can be used. For example, an anti-EGFR antibody can be used as the primary antibody when EGFR (expressed protein) is the biological substance of interest, and an anti-HER2 antibody can be used as the primary antibody when HER2 is the biological substance of interest.

As a secondary antibody, an antibody (IgG) which specifically recognizes and binds to the primary antibody as an antigen can be used.

Both of the primary antibody and the secondary antibody may be polyclonal antibodies; however, from the standpoint of the stability of quantification, they are preferably monoclonal antibodies. The kind of the animal (immune animal) used for producing the antibodies is not particularly restricted, and the animal may be selected from mice, rats, guinea pigs, rabbits, goats, sheep and the like as in conventional cases.

The primary antibody does not have to be a natural full-length antibody and may be an antibody fragment or derivative, as long as it is capable of specifically recognizing and binding to a specific biological substance (antigen). That is, the term "antibody" used herein encompasses not only full-length antibodies but also antibody fragments such as Fab, F(ab) '2, Fv, and scFv as well as derivatives such as chimeric antibodies (e.g., humanized antibodies) and multifunctional antibodies.

<Fluorescent Nanoparticles>

The fluorescent nanoparticles used in the present invention are preferably "fluorescent substance-integrated nanoparticles" which are capable of emitting fluorescence with an intensity sufficient for indicating each molecule of the biological substance of interest as a bright spot.

The term "fluorescent substance" used herein refers to a substance whose electrons are excited when the substance is irradiated with an electromagnetic wave of a prescribed wavelength (X-ray, UV radiation or visible light) and absorbs the energy thereof and which releases an excess energy in the form of an electromagnetic wave during the transition from an excited state to the ground state, namely a substance which emits "fluorescence", and the substance can be directly or indirectly bound with the secondary antibody. Further, the term "fluorescence" has a broad meaning and encompasses phosphorescence which has a long emission lifetime sustaining the emission even after the irradiation with an electromagnetic wave for excitation is terminated; and fluorescence in a narrow sense, which has a short emission lifetime.

<Fluorescent Substance-integrated Nanoparticles>

The fluorescent substance-integrated nanoparticles in the present invention are nano-sized particles having a structure in which a particle made of an organic or inorganic substance is a matrix and a plurality of fluorescent substances (e.g., fluorescent dyes) are encapsulated in the matrix and/or adsorbed on the surface of the matrix. In this case, it is preferred that the matrix (e.g., a resin) and the fluorescent substances each comprise a substituent or moiety having an opposite electric charge and that an electrostatic interaction occur therebetween.

Among matrices that can constitute the fluorescent substance-integrated nanoparticles, examples of an organic substance include resins that are generally classified into thermosetting resins, such as melamine resins, urea resins, aniline resins, guanamine resin, phenolic resins, xylene resins and furan resins; resins that are generally classified into thermoplastic resins, such as styrene resins, acrylic resins, acrylonitrile resins, AS resins (acrylonitrile-styrene copolymers) and ASA resins (acrylonitrile-styrene-methyl acrylate copolymers) ; other resins such as polylactic acids; and polysaccharides, and examples of an inorganic substance include silica and glass.

• Semiconductor-integrated Nanoparticles

Semiconductor-integrated nanoparticles have a structure in which semiconductor nanoparticles as phosphors are encapsulated in the above-described matrix and/or adsorbed on the surface of the matrix. The material constituting the semiconductor nanoparticles is not particularly restricted and may be a material containing a Group II-VI compound, a Group III-V compound or a Group IV element, examples of which include CdSe, CdS, CdTe, ZnSe, ZnS, ZnTe, InP, InN, InAs, InGaP, GaP, GaAs, Si, and Ge. When the semiconductor is encapsulated in the matrix, the semiconductor may or may not be chemically bound with the matrix itself, as long as the semiconductor is dispersed in the matrix.

• Fluorescent Dye-integrated Nanoparticles

Fluorescent dye-integrated nanoparticles have a structure in which a fluorescent dye is encapsulated in the above-described matrix and/or adsorbed on the surface of the matrix. The fluorescent dye is not particularly restricted, and examples thereof include rhodamine-based dye molecules, squarylium-based dye molecules, cyanine-based dye molecules, aromatic ring-containing dye molecules, oxazine-based dye molecules, carbopyronine-based dye molecules, and pyrromethene-based dye molecules. Alternatively, for example, Alexa Fluor (registered trademark, manufactured by Invitrogen Corp.)-based dye molecules, BODIPY (registered trademark, manufactured by Invitrogen Corp.)-based dye molecules, Cy (registered trademark, manufactured by GE Healthcare)-based dye molecules, DY (registered trademark, manufactured by Dyomics GmbH)-based dye molecules, HiLyte (registered trademark, manufactured by AnaSpec Inc.)-based dye molecules, DyLight (registered trademark, manufactured by Thermo Fisher Scientific K.K.)-based dye molecules, ATTO (registered trademark, manufactured by ATTO-TEC GmbH)-based dye molecules, and MFP (registered trademark, manufactured by Mobitec Co., Ltd.)-based dye molecules can be used as well. The generic names of these dye molecules are assigned based on the main structure (skeleton) or registered trademark of the respective compounds; therefore, those of ordinary skill in the art should be able to properly understand the scope of fluorescent dyes belonging to the respective generic names without having to bear undue trial and error. Further, when the fluorescent dye is encapsulated in the matrix, the fluorescent dye may or may not be chemically bound with the matrix itself, as long as the fluorescent dye is dispersed inside the matrix.

Fluorescent substance-integrated nanoparticles can be produced in accordance with a known method (see, for example, JP 2013-57937 A). More specifically, for example, fluorescent substance-containing silica particles in which silica is used as a matrix and a fluorescent substance is encapsulated therein can be produced by adding dropwise a solution, in which inorganic semiconductor nanoparticles, a fluorescent substance such as an organic fluorescent dye and a silica precursor such as tetraethoxysilane are dissolved, to a solution in which ethanol and ammonia are dissolved, and subsequently hydrolyzing the silica precursor. Meanwhile, fluorescent substance-containing resin particles in which a resin is used as a matrix and a fluorescent substance is adsorbed on the surface of the resin particles or encapsulated in the resin particles can be produced by preparing in advance a solution of the resin or a dispersion of fine particles of the resin, adding thereto inorganic semiconductor nanoparticles and a fluorescent substance such as an organic fluorescent dye, and subsequently stirring the resultant. Alternatively, fluorescent substance-containing resin particles can also be produced by adding a fluorescent dye to a solution of a resin material and then allowing polymerization reaction to proceed. For example, in cases where a thermosetting resin such as a melamine resin is used as a matrix resin, organic fluorescent dye-containing resin particles can be produced by heating a reaction mixture, which contains a raw material of the resin (a monomer, an oligomer or a prepolymer, such as methylolmelamine obtained by condensation of melamine and formaldehyde), an organic fluorescent dye, and preferably further a surfactant and a polymerization reaction accelerator (e.g. an acid), and thereby allowing polymerization reaction to proceed by an emulsion polymerization method. Further, in cases where a thermoplastic resin such as a styrene-based copolymer is used as a matrix resin, organic fluorescent dye-containing resin particles can be produced by heating a reaction mixture, which contains a raw material of the resin, an organic fluorescent dye (as a resin material monomer, a monomer bound with an organic fluorescent dye through a covalent bond or the like in advance may also be used) and a polymerization initiator (e.g. benzoyl peroxide or azobis-isobutyronitrile), and thereby allowing polymerization reaction to proceed by a radical polymerization method or an ionic polymerization method.

Examples of the fluorescent substance to be contained in such fluorescent substance-integrated nanoparticles include, in addition to the above-described semiconductor nanoparticles and fluorescent dyes, "long-afterglow phos-

phors" that comprise $Y_2O_3$, $Zn_2SiO_4$ or the like as a matrix and $Mn^{2+}$, $Eu^{3+}$ or the like as an activator.

The average particles size of the fluorescent substance-integrated nanoparticles (particularly, fluorescent dye-containing resin particles obtained by such a production method as described above) is not particularly restricted as long as it is suitable for immunostaining (or gene staining) of a pathological specimen; however, taking into consideration, for example, the ease of detecting the fluorescent substance-integrated nanoparticles as bright spots, the average particle size is usually 10 to 500 nm, preferably 50 to 200 nm. Further, the variation coefficient, which represents the variation in the particle size, is usually 20% or less, preferably 5 to 15%. Fluorescent substance-integrated nanoparticles satisfying these conditions can be produced by adjusting the production conditions. For example, in the production of such fluorescent substance-integrated nanoparticles by an emulsion polymerization method, the particle size can be adjusted by changing the amount of a surfactant to be added and, generally speaking, when the amount of the surfactant is relatively large with respect to the amount of the parent materials of the fluorescent substance-integrated nanoparticles, the particle size tends to be small, whereas when the amount of the surfactant is relatively small, the particle size tends to be large.

The size of a fluorescent substance-integrated nanoparticle can be determined by taking an electron micrograph thereof using a scanning electron microscope (SEM), measuring the cross-sectional area of the fluorescent substance-integrated nanoparticle and then calculating the particle size as the diameter of a circle corresponding to the thus measured cross-sectional area with an assumption that the cross-sectional shape is a circle. With regard to the average particle size and variation coefficient of a group of plural fluorescent substance-integrated nanoparticles, after determining the particle size for a sufficient number (e.g., 1,000) of the * fluorescent substance-integrated nanoparticles in the above-described manner, the average particle size is calculated as the arithmetic mean of the thus obtained values, and the variation coefficient is calculated using the following equation:

```
100 × (standard deviation of particle size)/(average particle
size).
```

<Constitution of Immunostaining Agent>

As described above, the fluorescent nanoparticles contained in the immunostaining agent used for fluorescent labeling of a biological substance of interest are preferably "fluorescent substance-integrated nanoparticles". In order to improve the fluorescent labeling efficiency and to thereby minimizes the lapse of time that leads to degradation of fluorescence, as the immunostaining agent, it is preferred to use a complex in which the primary antibody and a phosphor are linked indirectly, that is, through a non-covalent bond formed by utilizing an antigen-antibody reaction, an avidin-biotin reaction or the like.

One example of the immunostaining agent in which a probe and fluorescent nanoparticles are linked indirectly is a complex of [primary antibody for the biological substance of interest]...[antibody (secondary antibody) for the primary antibody]-[fluorescent nanoparticle (fluorescent substance-integrated nanoparticle)], wherein "..." represents a bond formed by an antigen-antibody reaction. The mode of the bond represented by "-" is not particularly restricted, and examples thereof include a covalent bond, an ionic bond, a hydrogen bond, a coordinate bond, physical adsorption, and chemical adsorption. As required, the bond may be formed via a linker molecule and, for example, a silane coupling agent which is a compound widely used for binding an inorganic substance with an organic substance can be employed. This silane coupling agent is a compound which has an alkoxysilyl group yielding a silanol group on hydrolysis at one end of the molecule and a functional group, such as a carboxyl group, an amino group, an epoxy group or an aldehyde group, at the other end, and binds with an inorganic substance via the oxygen atom of the silanol group. Specific examples of the silane coupling agent include mercaptopropyltriethoxysilane, glycidoxypropyltriethoxysilane, aminopropyltriethoxysilane, and polyethylene glycol chain-containing silane coupling agents (e.g., PEG-silane no. SIM6492.7, manufactured by Gelest, Inc.). These silane coupling agents may be used in a combination of two or more thereof.

The reaction between the fluorescent substance-containing nanoparticles and the silane coupling agent can be carried out by a known method. For example, the resulting fluorescent substance-containing silica nanoparticles are dispersed in pure water, and aminopropyltriethoxysilane is subsequently added thereto and allowed to react at room temperature for 12 hours. After the completion of the reaction, fluorescent substance-containing silica nanoparticles whose surfaces have been modified with aminopropyl groups can be obtained through centrifugation or filtration. Subsequently, by allowing amino groups to react with a carboxyl group of an antibody, the antibody can be bound to the fluorescent substance-containing silica nanoparticles through amide bonds. As required, a condensation agent such as EDC
[1-ethyl-3-[3-dimethylaminopropyl]carbodiimide hydrochloride, manufactured by Pierce] can also be used.

As required, a linker compound which has a site capable of directly binding to an organic molecule-modified fluorescent substance-containing silica nanoparticle and a site capable of binding to a molecular target substance can be used. For example, when sulfo-SMCC [sulfosuccinimidyl-4-[N-maleimidomethyl]cyclohexane-1-carboxy late, manufactured by Pierce] which has both a site that selectively reacts with an amino group and a site that selectively reacts with a mercapto group is used, the amino groups of the fluorescent substance-containing silica nanoparticles modified with aminopropyltriethoxysilane and the mercapto group of the antibody are bound with each other, whereby fluorescent substance-containing silica nanoparticles bound with the antibody can be obtained.

For binding of a biological substance-recognizing site (a site capable of capable of specifically recognizing a biological substance, e.g., biotin, avidin or an antibody) to fluorescent substance-containing polystyrene nanoparticles, the same procedure can be applied regardless of whether the fluorescent substance is a fluorescent dye or a semiconductor nanoparticle. That is, by impregnating polystyrene nanoparticles having a functional group, such as an amino group, with semiconductor nanoparticles or an organic fluorescent dye, fluorescent substance-containing polystyrene nanoparticles having the functional group can be obtained and, by using EDC or sulfo-SMCC in the subsequent process, fluorescent substance-containing polystyrene nanoparticles bound with an antibody can be prepared.

Another example of the immunostaining agent in which a probe and a phosphor are linked indirectly is a complex composed of three molecules that are linked together by a mode of [primary antibody for the biological substance of interest] ... [antibody (secondary antibody) for the primary antibody] - [biotin] / [avidin] - [phosphor (fluorescent nanoparticle)] (wherein, "..." represents a bond formed by an antigen-antibody reaction; "-" represents a covalent bond which may be formed via a linker molecule as required; and "/" represents a bond formed by an avidin-biotin reaction).

A secondary antibody-biotin conjugate (biotin-modified secondary antibody) can be prepared using, for example, a commercially available biotin labeling reagent (kit) based on a known method by which biotin can be bound to a desired antibody (protein). Alternatively, if a biotin-modified secondary antibody in which biotin has been bound to a desired antibody in advance is commercially available, such a secondary antibody may be utilized as well.

A fluorescent nanoparticle-avidin conjugate (avidin-modif ied phosphor) can also be prepared using, for example, a commercially available avidin labeling reagent (kit) based on a known method by which avidin can be bound to a phosphor. In this case, avidin may be of a modified type, such as streptavidin or NeutrAvidin, which exhibits a higher binding strength with biotin than avidin.

Specific examples of a method of preparing a phosphor-avidin conjugate include the followings.

When the fluorescent nanoparticles are fluorescent substance-integrated nanoparticles containing a resin as its matrix, a functional group of the resin and a functional group of avidin (protein) can be bound with each other through, as required, a linker molecule such as PEG that has functional groups at both ends of the molecule.

For example, when the resin is a melamine resin, its functional group such as an amino group can be utilized and, when the resin is an acrylic resin, a styrene resin or the like, a monomer having a functional group (e.g., an epoxy group) in the side chain may be copolymerized with the resin to utilize the functional group itself or a functional group converted therefrom (e.g., an amino group generated by a reaction with aqueous ammonia), or these functional groups may be utilized to introduce other functional group(s). Further, when the fluorescent nanoparticles are fluorescent substance-integrated nanoparticles containing silica as its matrix or inorganic semiconductor nanoparticles, a desired functional group can be introduced by surface modification with a silane coupling agent and, for example, an amino group can be introduced by using aminopropyltrimethoxysilane.

Meanwhile, with regard to avidin, a thiol group can be introduced to avidin by allowing the amino group of avidin to react with, for example, N-succinimidyl-S-acetylthioacetate (SATA).

Further, an amino group-containing phosphor and the thiol-introduced avidin can be linked with each other by utilizing a cross-linker reagent which has N-hydroxysuccinimide (NHS) ester that is reactive with an amino group and a maleimide group that is reactive with a thiol group on the respective ends of a polyethylene glycol (PEG) chain.

A secondary antibody-fluorescent nanoparticle conjugate can be prepared using, for example, a commercially available fluorescent labeling reagent (kit) based on a known method by which a desired fluorescent dye can be bound to a desired antibody (protein). Alternatively, if a secondary antibody-fluorescent nanoparticle conjugate in which desired fluorescent nanoparticles have been bound to a desired antibody in advance is commercially available, such a conjugate may be utilized as well.

- Method of Staining Tissue Section -

(Immunostaining Method)

One examples of the staining method employed in the present invention will now be described. The method of preparing a tissue section (the term "tissue section" may be hereinafter simply referred to as "section" and used as a term that encompasses such sections as pathological sections) to which this staining method can be applied is not particularly restricted, and a tissue section prepared by a known procedure can be used.

(1. Sample Preparation Step)

(1-1. Deparaffinization Treatment)

The subject section is immersed in xylene contained in a vessel to remove paraffin. The temperature of this process is not particularly restricted and may be room temperature. The immersion time is preferably 3 minutes or longer but not longer than 30 minutes. If necessary, xylene may be replaced anew during the immersion.

Then, the section is immersed in ethanol contained in a vessel to remove xylene. The temperature of this process is not particularly restricted and may be room temperature. The immersion time is preferably 3 minutes or longer but not longer than 30 minutes. If necessary, ethanol may be replaced anew during the immersion.

The section is further immersed in water contained in a vessel to remove ethanol. The temperature of this process is not particularly restricted and may be room temperature. The immersion time is preferably 3 minutes or longer but not longer than 30 minutes. If necessary, water may be replaced anew during the immersion.

(1-2. Retrieval Treatment)

In accordance with a known method, a biological substance of interest to be stained is retrieved. The retrieval conditions are not particularly defined here; however, as a retrieval liquid, for example, 0.01 M citrate buffer (pH 6.0), 1 mM EDTA solution (pH 8.0), 5% urea or 0.1 M Tris-HCl buffer can be used. As a heating equipment, for example, an autoclave, a microwave oven, a pressure cooker or a water bath can be used. The temperature is not particularly restricted, and the retrieval may be performed at room temperature. The heating can be performed at a temperature of 50 to 130°C for a period of 5 to 30 minutes.

Then, the thus retrieved section is immersed and washed in PBS contained in a vessel. The temperature of this process is not particularly restricted and may be room temperature. The immersion time is preferably 3 minutes or longer but not longer than 30 minutes. If necessary, PBS may be replaced anew during the immersion.

(2. Immunostaining Step)

In the immunostaining step, in order to stain the biological substance of interest, fluorescent nanoparticles having a site capable of directly or indirectly binding to the biological substance of interest are dispersed in the fluorescent nanoparticle diluent of the present invention, and the resulting dispersion is place on the tissue section to allow the fluorescent nanoparticles to react with the biological substance of interest. The immunofluorescent staining solution used in the immunostaining step and the fluorescent nanoparticle diluent and other components used for the preparation thereof are as described above, and the immunofluorescent staining solution can be prepared in advance before the present step.

For example, when the immunostaining agent is a complex of [primary antibody (probe)]...[secondary antibody]-[biotin]/[avidin]-[fluorescent substance-containing nanoparticle (phosphor)] (wherein, "..." represents a bond formed by an antigen-antibody reaction; "-" represents a covalent bond which may be formed via a linker molecule as required; and "/" represents a bond formed by an avidin-biotin reaction), the processes of first immersing the pathological specimen in a primary antibody solution (primary reaction treatment), subsequently immersing the pathological specimen in a secondary antibody-biotin conjugate solution (secondary reaction treatment), and lastly immersing the tissue section, which is the pathological specimen, in a solution (immunofluorescent staining solution) obtained by dispersing avidin-fluorescent dye-containing nanoparticles in the fluorescent nanoparticle diluent of the present invention (fluorescent labeling treatment) may be performed.

The conditions for performing the immunostaining step, such as the temperature and time of the immersion of the tissue section, which is the pathological specimen, in a prescribed solution (reagent) in each of the primary and secondary reaction treatments and the fluorescent labeling treatment, can be adjusted as appropriate in accordance with a conventional immunostaining method such that appropriate signals can be obtained.

The temperature of the immunostaining step is not particularly restricted, and the immunostaining step can be performed at room temperature. The reaction time is preferably 30 minutes or longer but not longer than 24 hours.

Prior to the above-described primary reaction treatment, it is preferred to add drops of a known blocking agent such as BSA-containing PBS or a surfactant such as Tween 20 to the tissue section. In the present invention, even when such a treatment of adding a blocking agent (blocking treatment) is performed prior to the primary reaction treatment, incorporation of the prescribed two kinds of proteins into the immunofluorescent staining solution (or the fluorescent nanoparticle diluent used for the preparation thereof) at the prescribed amounts allows the actions and effects of the present invention, such as reduction of the background noise, to be exerted.

(3. Sample Post-treatment Step)

After the completion of the immunostaining step, the pathological specimen is preferably subjected to treatments, such as fixation-dehydration, clearing and mounting, such that the tissue section is made suitable for observation.

The fixation-dehydration treatment can be performed by immersing the pathological specimen in a fixation liquid (a cross-linking agent such as formalin, paraformaldehyde, glutaraldehyde, acetone, ethanol, or methanol). The clearing can be performed by immersing the thus fixed and dehydrated pathological specimen in a clearing liquid (e.g. xylene). The mounting treatment can be performed by immersing the thus cleared pathological specimen in a mounting medium. The conditions for performing these treatments, such as the temperature and time of immersing the pathological specimen in each prescribed treatment liquid, can be adjusted as appropriate in accordance with a conventional immunostaining method such that appropriate signals can be obtained.

(4. Optional Step)

In the present invention, if necessary, a staining step for morphological observation can be incorporated so that the morphology of cells, tissues, organs and the like can be observed in a bright field. The staining step for morphological observation can be performed in accordance with a conventional method. For the morphological observation of a tissue sample, eosin staining which stains cytoplasm, interstitial tissues, various fibers, erythrocytes and keratinocytes in red to dark red is typically employed. Further, hematoxylin staining which stains cell nuclei, calcareous parts, cartilaginous tissues, bacteria and mucus in livid to light blue is also typically employed (a method of simultaneously performing these two staining processes is known as "hematoxylin-eosin staining" (HE staining)). In cases where the staining step for morphological observation is incorporated, it may be performed after or before the immunostaining step.

(5. Evaluation Step)

(5-1. Observation and Image-capturing)

In the observation and image-capturing step, in the same visual field under a microscope at a desired magnification, the pathological specimen is irradiated with excitation lights corresponding to the respective phosphors with which the biological substance of interest was fluorescently labeled in the immunostaining step, and immunostained images produced by the fluorescence emitted from the phosphors are observed and captured. These excitation lights can be irradiated using, for example, a laser light source installed in a fluorescence microscope and, as required, an optical filter for excitation light which selectively transmits a prescribed wavelength. The immunostained images can be captured using, for example, a digital camera mounted on the fluorescence microscope. In the process of capturing the immunostained images, by using, as required, an optical filter for fluorescence which selectively transmits a prescribed wavelength, immunostained images including only the desired fluorescence, from which undesired fluorescence, noise-causing exciting light and other lights are excluded, can be obtained.

(5-2. Image Processing and Signal Measurement)

In the image processing and measurement step, on the immunostained images captured for the biological substance of interest, the fluorescently labeled signals corresponding to the biological substance of interest are measured based on the results of image processing, and the fluorescently labeled signals corresponding to the biological substance of interest that exist in the cell membrane region are identified.

The fluorescently labeled signals are preferably measured in terms of the number of fluorescent bright spots.

Examples of software that can be used for the image processing include "ImageJ" (open source). The use of such an image processing software enables to perform a process of extracting bright spots of a prescribed wavelength (color) from the immunostained images and determining the total brightness of the bright spots and a process of measuring the number of bright spots having a brightness of not less than a prescribed value, particularly those processes for carrying out the above-described embodiments, in a semi-automatic and prompt manner.

Further, since the bright spots are each derived from a single fluorescent nanoparticle, they have a constant size and can be observed and recognized under a microscope. Those signals that are larger than a certain size (e.g., the average size of the observed fluorescent nanoparticles) are judged as aggregated bright spots. The bright spots and the aggregated bright spots can be semi-automatically and promptly distinguished using a software.

(Gene Staining Method)

In addition to the above-described immunostaining method, the present invention can also be applied to a method of specifically staining a gene with fluorescent nanoparticles (e.g., FISH method) . That is, the gene staining method according to the present invention is a method of staining a gene with a gene staining solution which comprises: the above-described fluorescent nanoparticle diluent; a probe; and fluorescent nanoparticles that are capable of binding to or bound with the probe.

(Probe)

The probe is, for example, a probe of a nucleic acid which specifically binds to a gene encoding a biomolecule specifically expressed in a disease (e.g., cancer), such as a gene encoding the above-described biological substance of interest.

The probe is, as described above, a nucleic acid molecule which is associated with a specific disease and has a sequence containing a part or the entirety of a specific region on a chromosome (probe sequence). Examples of the nucleic acid include naturally-occurring nucleic acids such as DNAs and RNAs (e.g., mRNA, tRNA, miRNA, siRNA and non-cording-RNA), and artificial nucleic acids such as PNAs and LNAs (or BNAs: Bridged Nucleic Acids). Accordingly, the nucleic acid molecule is not restricted as long as it is capable of forming a strand complementary to a nucleic acid sequence on the chromosome. The nucleic acid molecule may be a natural nucleic acid, an artificial nucleic acid, or a nucleic acid molecule in which a natural nucleic acid and an artificial nucleic acid are ligated.

(Preparation of Probe)

The probe for a gene of interest can be prepared in accordance with a known method, or it may be obtained as a commercial product. Taking into consideration the conditions for hybridization, the probe can be prepared to have such a base length, base sequence and GC content that gives appropriate stringency.

(Binding of Probe and Fluorescent Nanoparticles)

The binding between the probe and the fluorescent nanoparticles is not particularly restricted as long as it does not cause a problem in the gene staining method (e.g., FISH), and the binding can be achieved by various modes. The probe and the fluorescent nanoparticles may be bound by either a direct binding method where the fluorescent nanoparticles are directly bound to the probe or an indirect binding method where the probe and the fluorescent nanoparticles are bound via bonds formed between biomolecules.

<Direct Binding Method>

Examples of the direct binding method include a method of binding a nucleic acid molecule and fluorescent nanoparticles by substituting a hydroxyl group of phosphoric acid bound to the ribose C5 position at the 5'-end of the nucleic acid molecule or a hydroxyl group bound to the ribose C1 position at the 3'-end of the nucleic acid molecule with a thiol group (SH group) using a known thiol group-introducing reagent and subsequently allowing this nucleic acid molecule to react with the fluorescent nanoparticles labeled with maleimide. Such a direct binding method can be preferably performed using a kit manufactured by Vector Laboratories, Inc., such as "5' EndTag™ Nucleic Acid Labeling System" or "3' EndTag DNA Labeling System".

As another method, maleimide group-modified fluorescent nanoparticles are allowed to react with a thiol-11-dUTP solution to obtain fluorescent nanoparticles bound with dUTP, and the thus obtained fluorescent nanoparticles bound with dUTP are subsequently incorporated into a nucleic acid molecule by a nick translation method, whereby the phosphor-integrated nanoparticles can be directly bound to the nucleic acid molecule.

<Indirect Binding Method>

The indirect binding method is a method of binding a probe and fluorescent nanoparticles via specific bonds formed between biomolecules (first and second binding group molecules) . As for the bonds formed between biomolecules, examples of the combination of the first and second biomolecules include an avidin-biotin binding system and a hapten-anti-hapten binding system.

Examples of a method of preparing DNA labeled with the first binding group molecule (e.g., biotin) include a method in which a specific base (e.g., thymine (T)) of a nucleic acid molecule is substituted with the first biomolecule (e.g., biotin) -labeled nucleotide (e.g., biotin-16-dUTP) by nick translation and fluorescent nanoparticles having (strep-to)avidin are subsequently bound to biotin of this probe.

Meanwhile, fluorescent nanoparticles modified with the second binding group molecule (e.g., streptavidin) can be prepared by, for example, as follows. A functional group is introduced to each of the fluorescent nanoparticles and the second binding group molecule using a functional group-introducing reagent, and the second biomolecule and the fluorescent nanoparticles are bound via bonds formed between their functional groups. In this case, a linker may exist between the functional groups. Examples of the combination of the functional groups include NHS ester group-amino group, and thiol group-maleimide group. Examples of the linker include EMCS (N-[ε-maleimidocaproyloxy]succinimide ester) (manufactured by former Thermo Fisher Scientific K.K.).

(Gene Staining)

For gene staining, (i) a method (direct method) in which, before staining, a probe and fluorescent nanoparticles are bound with each other to prepare a conjugate, and a gene to be detected is subsequently stained with the conjugate, or (ii) a method (indirect method) in which, after allowing a probe modified with the first binding group molecule (e.g., biotin) to hybridize (specifically bind) to a gene to be detected (e.g., HER2 gene) (primary reaction treatment), fluorescent nanoparticles modified with the second binding group molecule (e.g., avidin) are specifically and dynamically bound to the probe (secondary reaction treatment), can be employed.

(FISH Method)

The FISH method can be performed in accordance with the procedures and treatment conditions that are standard for the above-described various techniques. Generally, a tissue section-mounted specimen slide may be immersed in one or more reagents suitable for the FISH method under appropriate temperature and time conditions. The various reagents required for performing FISH according to the present invention can be prepared in accordance with a known method, or they may be obtained as commercial products.

In the FISH method, as sample pretreatments, a deparaffinization treatment, a pretreatment for FISH, an enzyme (protease) treatment, a fixation treatment and the like are performed. As staining treatments, staining treatments based on FISH method (FISH staining), namely, for example, a DNA modification treatment, a hybridization treatment and a post-hybridization treatment, as well as, usually, an additional nuclear staining treatment (using, for example, DAPI) are performed. As sample post-treatments, a solvent replacement treatment, a loading treatment (mounting treatment using a mounting medium), a protection treatment and, as required, a washing treatment and a dehydration treatment that are performed before the solvent replacement treatment, are performed.

The actions and effects of the present invention will now be described.

(1) As long as the fluorescent nanoparticle diluent used for performing immunostaining with fluorescent nanoparticles comprises 1 to 5%(W/W) of a protein having a molecular weight of 40,000 or higher and 1 to 3% (W/W) of a protein having a molecular weight of less than 40,000, by using the fluorescent nanoparticle diluent of the present invention to dilute fluorescent particles in immunofluorescent staining, non-specific adsorption of the fluorescent nanoparticles can be inhibited and the background noise observed at the time of detection can be reduced, so that the accuracy and the quantitative performance in the evaluation of a stained image can be improved.

(2) As long as the protein having a molecular weight of 40,000 or higher is BSA, an effect of stabilizing proteins other than BSA is exerted particularly when the above-described dilution is performed, which is preferred.

(3) As long as the protein having a molecular weight of less than 40,000 is casein, an effect of filling (closing) the gaps between the molecules of the protein having a molecular weight of 40,000 or higher that are adhered to the part to which proteins adsorb non-specifically (e.g., a portion of a section in the periphery of an antigen) can be preferably attained. Casein contains a water-insoluble oily component (hydrophobic component) coordinated outside each casein molecule and the casein molecules are thus likely to aggregate with each other; therefore, the casein molecules aggregate with each other to fill up the gaps in accordance with the shape and the size of each gap, and this enables to preferably inhibit the non-specific adsorption.

(4 and 5) As long as the ratio of $\kappa$-casein in the above-described casein is in a range of 0 to 10% (W/W) or the ratio of $\alpha$-casein and $\beta$-casein ($\alpha$-casein:$\beta$-casein) is 40:60 to 60:40 (taking the total amount of $\alpha$-casein and $\beta$-casein as 100), in the observation and image-capturing step and the image processing and measurement step that are performed after the immunostaining step, the background is further reduced and the evaluation can thus be carried out with superior quantitative performance.

(6) As long as the immunofluorescent staining kit comprises (i) the fluorescent nanoparticle diluent according to any one of the above (1) to (5) and (ii) fluorescent nanoparticle-containing immunostaining reagent, the above-described non-specific adsorption-inhibiting effect can be attained relatively easily by simply mixing the fluorescent nanoparticle diluent with the immunostaining reagent and using the resulting mixture for immunofluorescent staining.

(7) As long as the immunofluorescent staining solution comprises the fluorescent nanoparticle diluent according to any one of the above (1) to (5) and fluorescent nanoparticles, since an effect of inhibiting the non-specific adsorption of the fluorescent nanoparticles to the inner surface of a container in which the solution is retained and that of a pipette tip used for sucking and discharging the solution can be attained, the fluorescent nanoparticles are not unnecessarily wasted prior to the immunostaining step.

(8) As long as the immunofluorescent staining method comprises the immunostaining step using the above-described immunofluorescent staining solution, since the non-specific adsorption to a container, a pipette tip and the like can also be preferably inhibited, the fluorescence signals are enhanced that much.

(9) As long as the immunostaining step of the immunofluorescent staining method comprises the step of sequentially performing: a primary reaction treatment of specifically binding a primary antibody to a biological substance of interest; a secondary reaction treatment of specifically binding a biotin-bound secondary antibody to the primary antibody; and a treatment of diluting avidin-bound fluorescent nanoparticles with the fluorescent nanoparticle diluent according to any one of claims 1 to 5 and then fluorescently labeling the secondary antibody with the resulting solution, not only the non-specific adsorption of the fluorescent nanoparticles is inhibited but also non-specific adsorption of avidin bound to the fluorescent nanoparticles is inhibited, as a result of which the fluorescent labeling treatment utilizing the avidin-biotin binding system can be preferably performed.

(10) As long as the fluorescent nanoparticle diluent, which is used for performing a FISH method with fluorescent nanoparticles, comprises 1 to 5%(W/W) of a protein having a molecular weight of 40,000 or higher and 1 to 3% (W/W) of a protein having a molecular weight of less than 40,000, the non-specific adsorption of the fluorescent nanoparticles is inhibited, so that the noise is reduced and the detection signals are enhanced when gene detection is performed.

(11) As long as the protein having a molecular weight of 40,000 or higher is BSA, the effect described in the above (2) can also be attained in gene detection.

(12) As long as the protein having a molecular weight of less than 40,000 is casein, the effect described in the above (3) can also be attained in gene detection.

(13 and 14) As long as the $\kappa$-casein content in the above-described casein is 10%(W/W) or less, or the ratio of $\alpha$-casein and $\beta$-casein ($\alpha$-casein:$\beta$-casein) is 40:60 to 60:40 (taking the total amount of $\alpha$-casein and $\beta$-casein as 100), the effect described in the above (4 and 5) can also be attained in gene detection.

(15) As long as the gene staining kit comprises (i) the fluorescent nanoparticle diluent according to any one of the above (10) to (14) and (ii) fluorescent nanoparticle-containing gene staining reagent, the effect described in the above (6) can also be attained in gene detection.

(16) As long as the gene staining solution comprises the fluorescent nanoparticle diluent according to any one of the above (10) to (14) and fluorescent nanoparticles, the effect described in the above (7) can also be attained in gene detection.

(17) As long as the gene staining method comprises the gene staining step using the above-described gene staining solution, the effect described in the above (8) can be attained (the same effect can be attained not only for the fluorescence signals but also for the isotope signals).

(18) As long as the gene staining step of the gene staining method comprises the step of sequentially performing: a primary reaction treatment of specifically binding a first binding group molecule-containing probe to a gene to be detected; and a secondary reaction treatment of diluting fluorescent nanoparticles bound with a second binding group molecule, which specifically binds to the first binding group molecule, with the fluorescent nanoparticle diluent according to any one of the above (10) to (14) and then fluorescently labeling the probe specifically bound to the gene to be detected with the resulting solution, non-specific adsorption of the fluorescent nanoparticles that is caused by non-specific adsorption of the second binding group molecule bound to the fluorescent nanoparticles can be preferably inhibited.

Particularly, since fluorescent nanoparticles labeled with the second binding group molecule (e.g., avidin) are used, for example, when a solution of the fluorescent nanoparticles is stored in a tube such as an Eppendorf tube until the use thereof, there is a problem that the surfaces of the fluorescent nanoparticles and the portion of the second binding group molecule (e.g., avidin) non-specifically bind to the inner wall of the tube; however, according to the present invention, such non-specific adsorption can be inhibited, so that the fluorescent nanoparticles are not likely to be wasted.

Further, according to the present invention, in the process of linking fluorescent nanoparticles to a probe, since non-specific adsorption of the fluorescent nanoparticles to a tissue section is inhibited, noise is reduced and (fluorescence) signals are enhanced in the detection of a gene of interest.

EXAMPLES

[Preparation Example 1] Preparation of Biotin-modified Anti-rabbit IgG Antibody

In a 50 mM Tris solution, 50 $\mu$g of an anti-rabbit IgG antibody to be used as a secondary antibody was dissolved. To the resulting solution, a DTT (dithiothreitol) solution was added to a final concentration of 3 mM, and the resultant was mixed and allowed to react at 37°C for 30 minutes. Then, the thus obtained reaction solution was passed through a desalting column "Zeba Desalt Spin Columns" (manufactured by Thermo Fisher Scientific K.K., Cat.# 89882) to purify a DTT-reduced secondary antibody. An antibody solution was prepared by dissolving 200 μL of the whole amount of the thus purified antibody in a 50 mM Tris solution. Meanwhile, a linker reagent "Maleimide-PEG$_2$-Biotin" (manufactured by Thermo Fisher Scientific K.K., Product No. 21901) was adjusted with DMSO to a concentration of 0.4 mM. Then, 8.5 μL of this linker reagent solution was added to the antibody solution, and the resultant was mixed and allowed to react at 37°C for 30 minutes, whereby biotin was bound to the anti-rabbit IgG antibody via a PEG chain. The resulting reaction solution was purified through a desalting column. The absorbance of the thus desalted reaction solution was measured at a wavelength of 300 nm using a spectrophotometer ("F-7000", manufactured by Hitachi, Ltd.) to determine the amount of the protein (biotin-modified secondary antibody) contained in the reaction solution. The reaction solution was adjusted with a 50 mM Tris solution to have a biotin-modified secondary antibody concentration of 250 μg/mL, and the resulting solution was used as a biotin-modified secondary antibody solution (reagent II).

[Preparation Example 2] Preparation of Texas Red Dye-containing Melamine Resin Nanoparticles

After dissolving 2.5 mg of Texas Red dye molecule "Sulforhodamine 101" (manufactured by Sigma-Aldrich) in 22.5 mL of pure water, the resulting solution was stirred for 20 minutes using a hot stirrer with the temperature of the solution being maintained at 70°C. Then, 1.5 g of a melamine resin "Nikalac MX-035" (manufactured by Nippon Carbide Industries Co., Ltd.) was added to the solution, and the resultant was further stirred with heating for 5 minutes under the same conditions. To the thus heat-stirred solution, 100 μL of formic acid was added, and the resulting solution was stirred for 20 minutes with its temperature being maintained at 60°C, after which the solution was left to stand and allowed to cool to room temperature. The thus cooled solution was dispensed into a plurality of centrifuge tubes and centrifuged at 12,000 rpm for 20 minutes to allow the Texas Red dye-containing melamine resin nanoparticles contained as a mixture in the solution to precipitate. After removing the resulting supernatant, the precipitated particles were washed with ethanol and water. Thereafter, 1,000 of the thus obtained nanoparticles were observed under an SEM and their average particle size was measured as described above, as a result of which the average particle size was found to be 152 nm.

[Preparation Example 3] Preparation of Streptavidin-modified Texas Red dye-containing Melamine Resin Nanoparticles

First, 0.1 mg of the particles obtained in Preparation Example 2 was dispersed in 1.5 mL of ethanol, and 2 μL of aminopropyltrimethoxysilane LS-3150 (manufactured by Shin-Etsu Chemical Co., Ltd.) was added thereto. The resulting mixture was allowed to react for 8 hours to perform a surface amination treatment.
Then, the thus surface-aminated particles were adjusted with PBS (phosphate-buffered physiological saline) containing 2 mM of EDTA (ethylenediamine tetraacetic acid) to a concentration of 3 nM, and this solution was mixed with SM(PEG)$_{12}$ (succinimidyl-[(N-maleimidopropionamid)-dodecaethylene glycol]ester, manufactured by Thermo Fisher Scientific K.K.) to a final concentration of 10 mM and allowed to react for 1 hour. This mixture was centrifuged at 10,000 G for 20 minutes and the resulting supernatant was removed, after which PBS containing 2 mM of EDTA was added to disperse the precipitates, and the resulting dispersion was centrifuged again. The precipitates were washed three times by the same procedure to obtain fluorescent substance-containing melamine nanoparticle having a maleimide group at a terminal.
Meanwhile, streptavidin (manufactured by Wako Pure Chemical Industries, Ltd.) was subjected to a thiol group addition treatment with N-succinimidyl-S-acetylthioacetate (SATA) and subsequently filtered through a gel-filtration column to obtain a solution of streptavidin capable of binding to the fluorescent substance-containing melamine nanoparticles.
The thus obtained fluorescent substance-containing melamine nanoparticles and streptavidin were mixed in PBS containing 2 mM of EDTA and allowed to react for 1 hour at room temperature. Then, the reaction was terminated with an addition of 10 mM mercaptoethanol. After concentrating the resulting solution using a centrifugation filter, unreacted streptavidin and the like were removed using a purification gel-filtration column, whereby fluorescent substance-containing melamine nanoparticles bound with streptavidin were obtained.

[Preparation Example 4] Preparation of Melamine Resin Particles Containing CdSe/ZnS Semiconductor Nanoparticles Having Carboxylate Group as Surface-modifying Group

Under an argon flow, 2.9 g of stearic acid, 620 mg of n-tetradecyl phosphonic acid and 250 mg of cadmium oxide were added to 7.5 g of tri-n-octylphosphine oxide, and the resultant was heated to 370°C and mixed. After cooling the resulting mixture to 270°C, a solution prepared by dissolving 200 mg of selenium in 2.5 mL of tributyl phosphine was added thereto, and the resultant was dried under reduced pressure to obtain cadmium selenide (CdSe)-core semiconductor nanoparticles coated with tri-n-octylphosphine oxide.

Then, 15 g of tri-n-octylphosphine oxide was added to the thus obtained CdSe-core semiconductor nanoparticles and the resultant was heated, after which a solution prepared by dissolving 1.1 g of zinc diethyldithiocarbamate in 10 mL of trioctylphosphine was added thereto at 270°C, whereby a CdSe/ZnS semiconductor nanoparticle-containing dispersion was obtained.

The thus obtained dispersion was added to decane such that the CdSe/ZnS semiconductor nanoparticles were dispersed at a concentration of 5% by mass. Surface modification was performed by adding 0.5 mL of sodium propionate to 10 μL of the resulting dispersion and stirring the resultant at room temperature. After adding 2. 5 mL of pure water to this reaction mixture, the resulting solution was stirred for 20 minutes using a hot stirrer with the temperature of the solution being maintained at 70°C. Then, 1.5 g of a melamine resin "Nikalac MX-035" (manufactured by Nippon Carbide Industries Co., Ltd.) was added to the solution, and the resultant was further stirred with heating for 5 minutes under the same conditions.

To the thus stirred solution, 100 μL of formic acid was added, and the resulting solution was stirred for 20 minutes with its temperature being maintained at 60°C, after which the solution was left to stand and allowed to cool to room temperature. The thus cooled solution was dispensed into a plurality of centrifuge tubes and centrifuged at 12,000 rpm for 20 minutes to allow the melamine resin nanoparticles contained as a mixture in the solution to precipitate. The resulting supernatant was removed, and the precipitated particles were subsequently washed with ethanol and water, whereby nanoparticles (quantum dot-containing melamine resin nanoparticles) having an average particle size of 150 nm were prepared.

[Preparation Example 5] Preparation of Streptavidin-modified Quantum Dot-containing Melamine Resin Nanoparticles

Streptavidin-modified quantum dot-containing melamine resin nanoparticles were obtained from 0.1 mg of the nanoparticles obtained in Preparation Example 4 in the same manner as in Preparation Example 3.

[Experimental Example 1]

(E1) Sample Preparation Step

A breast cancer tissue array slide (tissue section-mounted glass slide; br243, manufactured by US Biomax, Inc.) was purchased and, using Ventana I-VIEW PATHWAY HER2 (4B5) kit, the tissue array slide was stained with Ventana BenchMark ULTRA, and the HER2 3+ region (i.e., cancer cell region) and the interstitial cell region were morphologically identified by a DAB method.

This sample was deparaffinized and then washed for replacement with water. The thus washed tissue array slide was subjected to a 15-minute autoclave treatment at 121°C in 10 mM citrate buffer (pH 6.0), thereby performing an antigen retrieval treatment. After the retrieval treatment, the tissue array slide was washed with PBS and then subjected to a 1-hour blocking treatment with 1% BSA-containing PBS.

(E2) Immunostaining Step

(E2-1) Primary Reaction Treatment of Immunostaining

As a primary reaction treatment for the first immunostaining of a biological substance of interest HER2, a primary reaction treatment liquid containing an anti-HER2 rabbit monoclonal antibody "4B5" (manufactured by Ventana Medical Systems, Inc.) at a concentration of 0.05 nM was prepared using 1-W/W% BSA-containing PBS, and the sample prepared in the step (1) was immersed in this primary reaction treatment liquid and allowed to react overnight at 4°C.

(E2-2) Secondary Reaction Treatment of Immunostaining

A secondary reaction treatment liquid was prepared by further diluting the biotin-modified anti-rabbit IgG antibody solution prepared in Preparation Example 1 with 1-W/W% BSA-containing PBS to a concentration of 6 μg/mL. The sample subjected to the primary reaction treatment was washed with PBS and subsequently immersed in this secondary reaction treatment liquid and allowed to react at room temperature for 30 minutes.

(E2-3) Fluorescent Labeling Treatment of Immunostaining

Fluorescent labeling reaction treatment liquids were each prepared by diluting the streptavidin-modified Texas Red dye-containing melamine resin particles prepared in Preparation Example 3 to a concentration of 0.02 nM with the respective fluorescent nanoparticle diluents having different content ratios of caseins (composition, $\alpha$-casein (c6780,

manufactured by Sigma-Aldrich): 50 W/W%, $\beta$-casein (c6905, manufactured by Sigma-Aldrich) : 50 W/W%) and BSA as shown in Table 1. The sample subjected to the secondary reaction treatment was immersed in these fluorescent labeling treatment liquids and allowed to react at room temperature for 3 hours under neutral pH environment (pH 6.9 to 7.4).

(E3) Sample Post-treatment Step

The thus immunostained sample was subjected to a fixation-dehydration treatment where the sample was immersed in pure ethanol for 5 minutes four times. Subsequently, the sample was subjected to a clearing treatment where the sample was immersed in xylene for 5 minutes four times. Finally, the sample was subjected to a mounting treatment where a mounting medium "Entellan New" (manufactured by Merck KGaA) was placed on the sample and a cover glass was further set thereon, whereby a sample for observation was prepared.

(E4) Evaluation Step

(E4-1) Observation and Image-capturing Step

In this step, a fluorescence microscope "BX-53" (manufactured by Olympus Corporation) was used for irradiation of an excitation light and observation of emitted fluorescence, and a microscope digital camera "DP73" (manufactured by Olympus Corporation) attached to the fluorescence microscope was used for taking immunostained images ($\times$400).
First, the sample was irradiated with an excitation light to cause the Texas Red dye, which was used for the fluorescent labeling of the biological substance of interest HER2, to emit fluorescence, and an immunostained image in this state was photographed. In this process, the wavelength of the excitation light was set at 575 to 600 nm using an excitation light optical filter installed in the fluorescence microscope, and the wavelength of the fluorescence to be observed was set at 612 to 692 nm using a fluorescence optical filter. The intensity of the excitation light in the observation and image capturing under the fluorescence microscope was set such that an irradiation energy of 900 W/cm$^2$ was provided in the vicinity of the center of the visual field. The exposure time for the image capturing was adjusted in such a range that does not cause saturation of the image brightness, and it was set, for example, at 4,000 $\mu$sec.
After immunostained images were captured in a single visual field, the same operations were repeated in different visual fields to capture immunostained images in a total of five visual fields (first to fifth visual fields) for each sample.

(E4-2) Image Processing and Measurement Step

For the image processing in this step, an image processing software "ImageJ" (open source) was used.
In each immunostained image, among the bright sports representing the Texas Red dye-containing melamine resin particles with which HER2 was fluorescently labeled, ones having a brightness of not less than a prescribed value were counted.
The interstitial noise, the number of bright spots per cell of the HER2 3+ region (that is, cancer cell region) and the number of aggregated bright spots were measured. The results thereof are shown in Table 1. It is noted here that, since HER2 is not expressed in the interstitial cell region, those bright spots positioned inside the interstitial cells are non-specific signals, that is, noise. A large number of bright spots representing the interstitial noise means the occurrence of non-specific reactions in a large number; therefore, the number of such bright spots was used as an evaluation index of immunoreaction.

[Table 1]

| | | Casein α-casein 50% β-casein 50% | | | | | | |
| --- | --- | --- | --- | --- | --- | --- | --- | --- |
| | | 0.6% | 0.8% | 1.0% | 1.2% | 2.4% | 3.0% | 5.0% |
| BSA | 0.5% | A; × B; × C; ○ | A; × B; × C; ○ | A; × B; × C; ○ | A; × B; ○ C; ○ | A; × B; × C; ○ | A; ○ B; × C; × | A; ○ B; × C; ○ |
| | 0.75% | A; × B; × C; ○ | A; × B; × C; ○ | A; × B; × C; ○ | A; × B; ○ C; ○ | A; × B; × C; ○ | A; ○ B; × C; × | A; ○ B; × C; ○ |
| | 1.0% | A; × B; ○ C; ○ | A; × B; ○ C; ○ | A; ◎ | | | | A; ○ B; × C; ○ |
| | 3.0% | A; × B; ○ C; ○ | A; × B; ○ C; ○ | B; ○ | | | | A; ○ B; × C; ○ |
| | 5.0% | A; × B; ○ C; × | A; × B; ○ C; ○ | C; ○ | | | | A; ○ B; × C; ○ |
| | 7.5% | A; × B; ○ C; × | A; × B; ○ C; × | A; × B; ○ C; × | A; ○ B; ○ C; × | A; ○ B; ○ C; × | A; ○ B; ○ C; × | A; ○ B; × C; × |
| | 10.0% | A; × B; ○ C; × | A; × B; ○ C; × | A; × B; ○ C; × | A; × B; ○ C; × | A; ○ B; × C; × | A; ○ B; × C; × | A; ○ B; × C; × |
| | 12.0% | A; × B; × C; × | A; × B; × C; × | A; × B; ○ C; × | A; × B; ○ C; × | A; ○ B; ○ C; × | A; ○ B; × C; × | A; ○ B; × C; × |

(In Table 1 above, "%" means "W/W%". The same applies to Tables 2 to 4 below.)

A = Interstitial noise (Number of bright spots (the same applies below))

500 or more = ×

300 to less than 500 = ○

200 to less than 300 = ◎

Less than 200 = ☆

B = Number of bright spots per cell

Less than 10 = ×

10 or more = ○

C = Aggregated bright spots

3 or more = ×

3 or less = ○

[Experimental Example 2]

The interstitial noise, the number of bright spots per cell and the number of aggregated bright spots were measured by performing the sample preparation step, the immunostaining step and the evaluation step in the same manner as in Experimental Example 1, except that the composition of caseins contained in the solution used for dispersing the fluorescent nanoparticles in (E2-3) Fluorescent Labeling Treatment of Immunostaining was changed. The results thereof are shown in Tables 2 to 4. It is noted here that the content ratios of the respective caseins in the natural casein are: $\alpha$-casein : 50%, $\beta$-casein : 35%, $\kappa$-casein : 13%, and $\gamma$-casein : 2%.

[Table 2]

| | | Casein (natural) | | | |
|---|---|---|---|---|---|
| | | 1.0% | 1.2% | 2.4% | 3.0% |
| BSA | 1.0% | • Interstitial noise: 300 to less than 500 = ○ | | | |
| | 3.0% | • Number of bright spots per cell: 10 or more = ○ | | | |
| | 5.0% | • Aggregated bright spot: none = ○ | | | |

[Table 3]

| | | Casein ($\alpha$-casein: 45%, $\beta$-casein: 45%, $\kappa$-casein: 10%) | | | |
|---|---|---|---|---|---|
| | | 1.0% | 1.2% | 2.4% | 3.0% |
| BSA | 1.0% | • Interstitial noise: less than 200 = ☆ | | | |
| | 3.0% | • Number of bright spots per cell: 10 or more = ○ | | | |
| | 5.0% | • Aggregated bright spot: none = ○ | | | |

[Table 3A]

| | | Casein ($\alpha$-casein: 49.5%, $\beta$-casein: 49.5%, $\kappa$-casein: 1.0%) | | | |
|---|---|---|---|---|---|
| | | 1.0% | 1.2% | 2.4% | 3.0% |
| BSA | 1.0% | • Interstitial noise: less than 200 = ☆ | | | |
| | 3.0% | • Number of bright spots per cell: 10 or more = ○ | | | |
| | 5.0% | • Aggregated bright spot: none = ○ | | | |

[Table 4]

| | | Casein ($\alpha$-casein: 50%, $\beta$-casein: 50%, $\kappa$-casein: 0%) | | | |
|---|---|---|---|---|---|
| | | 1.0% | 1.2% | 2.4% | 3.0% |
| BSA | 1.0% | • Interstitial noise: 200 to less than 300 = ◎ | | | |
| | 3.0% | • Number of bright spots per cell: 10 or more = ○ | | | |
| | 5.0% | • Aggregated bright spot: none = ○ | | | |

(extracted from Table 1)

[Experimental Example 3]

The interstitial region was identified using Ventana I-VIEW PATHWAY HER2 (4B5) kit in the same manner as in (E1). Then, staining and evaluation were performed in the same manner as in (E2) to (E4), except that an anti-HER3 rabbit monoclonal antibody "SP71" (manufactured by Abnova Corporation) was used in place of the anti-HER2 rabbit monoclonal antibody "4B5".

Consequently, results similar to those of Experimental Example 2 were obtained.

[Table 5]

| | | Casein (natural) | | | |
|---|---|---|---|---|---|
| | | 1.0% | 1.2% | 2.4% | 3.0% |
| BSA | 1.0% | • Interstitial noise: 300 to less than 500 = ○ | | | |
| | 3.0% | • Number of bright spots per cell: 5 or more | | | |
| | 5.0% | • Aggregated bright spot: none = ○ | | | |

[Table 6]

| | | Casein ($\alpha$-casein: 45%, $\beta$-casein: 45%, $\kappa$-casein: 10%) | | | |
|---|---|---|---|---|---|
| | | 1.0% | 1.2% | 2.4% | 3.0% |
| BSA | 1.0% | • Interstitial noise: less than 200 = ☆ | | | |
| | 3.0% | • Number of bright spots per cell: 5 or more | | | |
| | 5.0% | • Aggregated bright spot: none = ○ | | | |

[Table 7]

| | | Casein ($\alpha$-casein: 49%, $\beta$-casein: 49%, $\kappa$-casein: 2%) | | | |
|---|---|---|---|---|---|
| | | 1.0% | 1.2% | 2.4% | 3.0% |
| BSA | 1.0% | • Interstitial noise: less than 200 = ☆ | | | |
| | 3.0% | • Number of bright spots per cell: 5 or more | | | |
| | 5.0% | • Aggregated bright spot: none = ○ | | | |

[Table 8]

| | | Casein ($\alpha$-casein: 50%, $\beta$-casein: 50%, $\kappa$-casein: 0%) | | | |
|---|---|---|---|---|---|
| | | 1.0% | 1.2% | 2.4% | 3.0% |
| BSA | 1.0% | • Interstitial noise: 200 to less than 300 = ◎ | | | |
| | 3.0% | • Number of bright spots per cell: 5 or more | | | |
| | 5.0% | • Aggregated bright spot: none = ○ | | | |

[Experimental Example 4]

A lung tissue array slide (tissue section-mounted glass slide; LC241b, manufactured by US Biomax, Inc.) was purchased, and the interstitial cell region was morphologically identified in the same manner as in (E1), except that an anti-PD-L1 rabbit monoclonal antibody "SP142" (manufactured by Spring Bioscience (SBS) Corporation) was used. Then, staining and evaluation were performed in the same manner as in (E2) to (E4), except that an anti-PD-L1 rabbit monoclonal antibody "SP142" (manufactured by Spring Bioscience (SBS) Corporation) was used in place of the anti-HER2 rabbit monoclonal antibody "4B5" and that the streptavidin-modified quantum dot-containing melamine resin nanoparticles prepared in Preparation Example 5 were used in place of the streptavidin-modified Texas Red dye-containing melamine resin particles. In (E), the wavelength of the irradiated excitation light was set at 415 to 455 nm using an excitation light optical filter ("QD655-C", manufactured by OPTO-LINE, Inc.) installed in the fluorescence microscope, and the wavelength of the fluorescence to be observed was set at 648 to 663 nm using a fluorescence optical filter.

Consequently, results similar to those of Experimental Example 2 were obtained.

[Table 9]

|  |  | Casein (natural) | | | |
|---|---|---|---|---|---|
|  |  | 1.0% | 1.2% | 2.4% | 3.0% |
| BSA | 1.0% | • Interstitial noise: 300 to less than 500 = ○ | | | |
|  | 3.0% | • Number of bright spots per cell: 5 or more | | | |
|  | 5.0% | • Aggregated bright spot: none = ○ | | | |

[Table 10]

|  |  | Casein ($\alpha$-casein: 47.5%, $\beta$-casein: 47.5%, $\kappa$-casein: 5.0%) | | | |
|---|---|---|---|---|---|
|  |  | 1.0% | 1.2% | 2.4% | 3.0% |
| BSA | 1.0% | • Interstitial noise: less than 200 = ☆ | | | |
|  | 3.0% | • Number of bright spots per cell: 5 or more | | | |
|  | 5.0% | • Aggregated bright spot: none = ○ | | | |

[Table 11]

|  |  | Casein ($\alpha$-casein: 49%, $\beta$-casein: 49%, $\kappa$-casein: 2%) | | | |
|---|---|---|---|---|---|
|  |  | 1.0% | 1.2% | 2.4% | 3.0% |
| BSA | 1.0% | • Interstitial noise: less than 200 = ☆ | | | |
|  | 3.0% | • Number of bright spots per cell: 5 or more | | | |
|  | 5.0% | • Aggregated bright spot: none = ○ | | | |

[Table 12]

|  |  | Casein ($\alpha$-casein: 50%, $\beta$-casein: 50%, $\kappa$-casein: 0%) | | | |
|---|---|---|---|---|---|
|  |  | 1.0% | 1.2% | 2.4% | 3.0% |
| BSA | 1.0% | • Interstitial noise: 200 to less than 300 = ◎ | | | |
|  | 3.0% | • Number of bright spots per cell: 5 or more | | | |
|  | 5.0% | • Aggregated bright spot: none = ○ | | | |

[Experimental Example 5]

The steps of Experimental Example 1 were performed in the same manner, except that the process (E1) of identifying the interstitial cell region was excluded. That is, a breast cancer tissue array slide (tissue section-mounted glass slide; br243, manufactured by US Biomax, Inc.) was purchased and, after deparaffinization thereof, the tissue array slide was washed for replacement with water. The thus washed tissue array slide was subjected to a 15-minute autoclave treatment at 121°C in 10 mM citrate buffer (pH 6.0), thereby performing an antigen retrieval treatment. After the retrieval treatment, the tissue array slide was washed with PBS and then subjected to a 1-hour blocking treatment with 1% BSA-containing PBS.

Immediately after performing (E2) in the same manner, hematoxylin staining was additionally performed for morphological observation, and (E3) and (E4) were subsequently performed in the same manner. As a result, it was confirmed that the number of bright spots per cell was 5 or more and that there was no aggregation of bright spots. The interstitial noise was not measured.

[Preparation Example 6] (Preparation of biotin-labeled BAC Probe)

In accordance with the method described in Cell Biochem. Biophys. 2006; 45(1):59, a nucleic acid molecule was prepared as described below. For 1 μg (5 μL) of a HER2-DNA clone (about 150 kbp) purchased from GSP Lab., Inc., dTTP of the HER2-DNA clone (nucleic acid molecule) was substituted with biotin-labeled dUTP by a nick translation method as described below in accordance with the protocol provided with a nick translation kit (product name: "GSP-Nick Translation Kit" K-015; manufactured by GSP Lab., Inc.).

Next, the resultant was allowed to react at 15°C for 4 hours, and the reaction was terminated by heating at 70°C for 10 minutes. Then, 25 μL of distilled water was added to the centrifuge tube. The resulting reaction solution of a biotin-labeled BAC probe was purified using a micro-spin column for nucleic acid purification ("MicroSpin S-200HR Column" manufactured by GE Healthcare, product number: "#27-5120-01"). To this solution, about 5.56 μL of 3 M sodium acetate solution (pH 5.2) and 150 μL of 100% ethanol were added, and the resultant was left to stand at -20°C for at least one hour and subsequently centrifuged at 4°C and 16,000 rpm for 10 minutes to form precipitates. Further, 500 μL of 70% ethanol was added, and the resultant was centrifuged at 4°C and 16,000 rpm for 1 minute, followed by removal of the resulting supernatant. Thereafter, 5 to 10 μL of distilled water was added to the thus formed precipitates and the precipitates were completely dissolved, thereby obtaining a solution of a biotin-labeled BAC probe having a final concentration of 1 μg/250 μL.

[Experimental Example 6]

The copy number of the HER2 gene was measured by FISH. As described below, FISH was carried out by performing deparaffinization, pretreatment of specimen slide, enzyme treatment, fixation of specimen, probe preparation, denaturation of DNA on specimen slide, hybridization, washing of glass slide and DAPI staining in the order mentioned.

[Deparaffinization]

A specimen slide of a HER2-positive staining control sample ("HER2-FISH Control Slide" manufactured by Pathology Institute Corp., code: PS-09006) was deparaffinized by sequentially performing the following treatments (1) to (4): (1) immersing the specimen slide in Hemo-De at normal temperature for 10 minutes; (2) immersing the specimen slide in fresh Hemo-De at normal temperature for 10 minutes, followed by three repetitions of the same operation; (3) immersing the specimen slide in 100% ethanol at room temperature for 5 minutes and washing the specimen slide twice, followed by dehydration; and (4) drying the specimen slide in the air or on a 45 to 50°C slide warmer.

[Pretreatment of Specimen Slide]

In order to improve the reachability of the DNA probe, the specimen slide was pretreated by sequentially performing the following operations (1) to (6) to remove the proteins of cell membranes and nuclear membranes: (1) treating the specimen slide with 0.2 mol/L HCl at room temperature for 20 minutes; (2) immersing the specimen slide in purified water for 3 minutes; (3) immersing the specimen slide in a washing buffer (2× SSC: standard saline citrate) for 3 minutes; (4) immersing the specimen slide in a 80°C pretreatment solution (1N NaSCN) for 30 minutes; (5) immersing the specimen slide in purified water for 1 minute; and (6) immersing the specimen slide in a washing buffer (2× SSC) for 5 minutes, followed by two repetitions of this immersion operation.

[Enzyme Treatment]

The thus pretreated specimen slide was subjected to an enzyme treatment by sequentially performing the following operations (1) to (4): (1) taking out the pretreated specimen slide and removing excess washing buffer by bringing the lower end of the glass slide into contact with a paper towel; (2) immersing the specimen slide in a protease solution heated to 37°C for 10 to 60 minutes, which immersion process is desirably performed with 25 mg protease (in 50 mL of 2,500 to 3,000 units/mg of pepsin/1M NaCl [pH 2.0] at 37°C for 60 minutes) so as to degrade the proteins, particularly collagen, of cell membranes and nuclear membranes; (3) immersing the specimen slide in a washing buffer for 5 minutes, followed by two repetitions of this operation; and (4) drying the specimen slide in the air or on a 45 to 50°C slide warmer for 2 to 5 minutes.

[Fixation of Specimen]

For fixation of the specimen, the pretreated specimen slide was subjected to the following treatments (1) to (3): (1) immersing the specimen slide in 10% neutral buffered formalin (4% paraformaldehyde-phosphate buffer" manufactured by Wako Pure Chemical Industries, Ltd., product number: 163-20145) at normal temperature for 10 minutes; (2) immersing the specimen slide in a washing buffer for 5 minutes, followed by two repetitions of the same operation; and (3) drying the specimen slide in the air or on a 45 to 50°C slide warmer for 2 to 5 minutes.

[Probe Preparation]

A solution of the DNA probe prepared in Preparation Example 6, which had been freeze-stored, was thawed back to room temperature, and the viscosity of the solution was sufficiently reduced to such a level at which an exact volume of the solution can be collected by pipette operation, after which the solution was mixed using a vortex mixer or the like.

[Denaturation of DNA on Specimen Slide]

For denaturation of DNA on the specimen slide, the thus specimen-fixed specimen slide was subjected to the following treatments (1) to (8): (1) prior to the preparation of the specimen slide, placing and preheating a moist box having a water-moistened paper towel on the bottom (a hermetic container whose side surfaces are taped with paper towel) in a 37°C incubator; (2) confirming that a denaturation solution (70% formamide/SSC [150 mM NaCl, 15 mM sodium citrate]) has a pH of 7.0 to 8.0 at normal temperature, placing the denaturation solution in a Coplin jar and heating the Coplin jar in a warm water bath until the solution temperature reaches $72°C \pm 1°C$ (leaving the Coplin jar in a $72 \pm 1°C$ warm water bath for at least 30 minutes); (3) marking a region on the back side of the specimen with a circle using a diamond pen or the like to clearly indicate a hybridization region; (4) immersing the specimen slide in the $72 \pm 1°C$ denaturation solution placed in the Coplin jar to denature the DNA on the specimen slide; (5) taking out the specimen slide from the denaturation solution using a forceps, immediately placing the specimen slide in 70% ethanol at room temperature, shaking the slide for removal of formamide and leaving the specimen slide immersed for 1 minute; (6) taking out the specimen slide from the 70% ethanol, placing the specimen slide in 85% ethanol, shaking the slide for removal of formamide and leaving the specimen immersed for 1 minute, followed by two repetitions of the same operations using 100% ethanol; (7) removing ethanol by bringing the lower end of the specimen glass slide into contact with a paper towel, and then wiping the back side of the glass slide with a paper towel; and (8) drying the specimen slide using a dryer or on a 45 to 50°C slide warmer for 2 to 5 minutes.

[Hybridization]

The thus denaturation-treated specimen slide was subjected to hybridization with 10 $\mu$L (10 to 50 ng) of the above-prepared DNA probe by sequentially performing the following treatments (1) to (3): (1) adding 10 $\mu$L of the above-prepared DNA probe to the hybridization region of the specimen slide and immediately placing a 22 mm $\times$ 22 mm cover glass over the probe to uniformly spread the probe while preventing air bubbles from entering the hybridization region; (2) sealing the cover glass with paper bond; and (3) placing the specimen slide in the previously heated moist box, placing the lid and then performing hybridization in a 37°C incubator for 14 to 18 hours.

[Washing of Glass Slide]

The thus hybridized specimen slide was washed by sequentially performing the following treatments (1) to (6): (1) placing a post-hybridization washing buffer ($2\times$ SSC/0.3% NP-40) in a Coplin jar and preheating the Coplin jar in a warm water bath until the temperature of the post-hybridization washing buffer reaches $72°C \pm 1°C$ (leaving the Coplin jar in a $72 \pm 1°C$ warm water bath for at least 30 minutes); (2) preparing another Coplin jar containing the post-hybridization washing buffer and maintaining it at normal temperature; (3) removing the paper bond seal using a forceps; (4) immersing the specimen slide in this post-hybridization washing buffer until the cover glass spontaneously comes off in the solution; (5) taking out the specimen slide from the solution, removing excess solution and then immersing the specimen slide in the post-hybridization washing buffer heated to $72 \pm 1°C$ for 2 minutes, which immersion treatment is desirably performed at a temperature of 73°C or lower for a period of 2 minutes or less; and (6) taking out the specimen slide from the Coplin jar and air-drying the specimen slide in shade (for example, in a closed drawer or on a shelf of a closed cabinet).

(Fluorescent Labeling Treatment of Probe)

To the biotin-labeled DNA probe bound with HER2 gene, the streptavidin-bound fluorescent substance-containing melamine nanoparticles of Preparation Example 3 were bound as follows.

The particles prepared in Preparation Example 3 were diluted with a diluent to a concentration of 0.02 nM, and 100 μL of the resultant was dropped onto the specimen slide to allow a binding reaction to take place at room temperature for 60 minutes. The specimen slide was subsequently washed three times by immersion in PBS for 5 minutes. A case where 1% BSA was used as the diluent was compared with a case where a mixture of caseins (composition, $\alpha$-casein (c6780, manufactured by Sigma-Aldrich): 50%, $\beta$-casein (c6905, manufactured by Sigma-Aldrich) : 50%) and BSA was used as the diluent.

(DAPI Staining)

DAPI staining was performed as follows. First, 10 μL of a DAPI counter-staining liquid was added to the hybridization region of the specimen slide. Next, after subjecting the specimen slide to hybridization, in order to count the number of cells, cell nuclei were stained by performing DAPI staining (2 μg/mL PBS) at 25°C for 10 minutes, and a cover glass was placed on the specimen slide. This specimen slide was stored in shade until signal measurement. As DAPI (4',6-diamidino-2-phenylindole dihydrochloride), "D1306" manufactured by Molecular Probes Inc. was used.

(Observation)

The specimen slide subjected to FISH as described above was observed in the following manner.

<Observation under Fluorescence Microscope>

As for observation under a fluorescence microscope, the section subjected to FISH as described above was observed (at ×600 magnification) under a fluorescence microscope Zeiss Imager (camera: MRm monochrome camera with cooling function, objective lens: ×60 oil immersion lens) at a magnification of ×600 to obtain fluorescence images (static fluorescence images) and to measure the number of bright spots.

As a result, comparing to the case where 1% BSA was used as the particle diluent, the number of bright spots (the number of bright spots per cell in the HER2 3+ region (that is, cancer cell region)) was found to be doubled in those cases where a mixed liquid containing 2.4% of a casein mixture (composition = $\alpha$-casein (c6780, manufactured by Sigma-Aldrich): 50%, $\beta$-casein (c6905, manufactured by Sigma-Aldrich) : 50%) and 1 to 5% of BSA was used.

The invention is particularly defined by the following items:

1. A fluorescent nanoparticle diluent, which is used for performing immunostaining with fluorescent nanoparticles and comprises:

    1 to 5% (W/W) of a protein having a molecular weight of 40,000 or higher; and
    1 to 3% (W/W) of a protein having a molecular weight of less than 40,000.

2. The fluorescent nanoparticle diluent according to item 1, wherein said protein having a molecular weight of 40,000 or higher is BSA.

3. The fluorescent nanoparticle diluent according to item 1 or 2, wherein said protein having a molecular weight of less than 40,000 is casein.

4. The fluorescent nanoparticle diluent according to item 3, wherein the ratio of $\kappa$-casein in said casein is 0 to 10%(W/W).

5. The fluorescent nanoparticle diluent according to item 3 or 4, wherein the ratio of $\alpha$-casein and $\beta$-casein ($\alpha$-casein:$\beta$-casein) contained in said casein is 40:60 to 60:40 (taking the total amount of $\alpha$-casein and $\beta$-casein as 100).

6. An immunofluorescent staining kit, comprising:

    (i) the fluorescent nanoparticle diluent according to any one of items 1 to 5; and
    (ii) a fluorescent nanoparticle-containing immunostaining reagent.

7. An immunofluorescent staining solution, comprising:

    the fluorescent nanoparticle diluent according to any one of items 1 to 5; and
    fluorescent nanoparticles.

8. An immunofluorescent staining method, comprising the immunostaining step using the immunofluorescent staining solution according to item 7.

9. The immunofluorescent staining method according to item 8, wherein said immunostaining step comprises the step of sequentially performing:

a primary reaction treatment of specifically binding a primary antibody to a biological substance of interest;
a secondary reaction treatment of specifically binding a biotin-bound secondary antibody to said primary antibody; and
a treatment of diluting avidin-bound fluorescent nanoparticles with the fluorescent nanoparticle diluent according to any one of items 1 to 5 and then fluorescently labeling said secondary antibody with the resulting solution.

10. A fluorescent nanoparticle diluent, which is used for performing a gene staining method with fluorescent nanoparticles and comprises:

1 to 5% (W/W) of a protein having a molecular weight of 40,000 or higher; and
1 to 3% (W/W) of a protein having a molecular weight of less than 40,000.

11. The fluorescent nanoparticle diluent according to item 10, wherein said protein having a molecular weight of 40,000 or higher is BSA.

12. The fluorescent nanoparticle diluent according to item 10 or 11, wherein said protein having a molecular weight of less than 40,000 is casein.

13. The fluorescent nanoparticle diluent according to item 12, wherein the $\kappa$-casein content in said casein is 10%(W/W) or less.

14. The fluorescent nanoparticle diluent according to item 12 or 13, wherein the ratio of $\alpha$-casein and $\beta$-casein ($\alpha$-casein:$\beta$-casein) contained in said casein is 40:60 to 60:40 (taking the total amount of $\alpha$-casein and $\beta$-casein as 100).

15. A gene staining kit, comprising:

(i) the fluorescent nanoparticle diluent according to any one of items 10 to 14; and
(ii) a fluorescent nanoparticle-containing gene staining reagent.

16. A gene staining solution, comprising:

the fluorescent nanoparticle diluent according to any one of items 10 to 14;
a probe; and
fluorescent nanoparticles that are capable of binding to or bound with said probe.

17. A gene staining method, comprising the gene staining step using the gene staining solution according to item 16.

18. The gene staining method according to item 17, wherein said gene staining step comprises the step of sequentially performing:

a primary reaction treatment of specifically binding a first binding group molecule-containing probe to a gene to be detected; and
a secondary reaction treatment of diluting fluorescent nanoparticles bound with a second binding group molecule, which specifically binds to said first binding group molecule, with the fluorescent nanoparticle diluent according to any one of items 10 to 14 and then fluorescently labeling said probe specifically bound to said gene to be detected with the resulting solution.

**Claims**

1. A fluorescent nanoparticle diluent, which is used for performing immunostaining with fluorescent nanoparticles and comprises:

1 to 5% (W/W) of a protein having a molecular weight of 40, 000 or higher; and
1 to 3% (W/W) of a protein having a molecular weight of less than 40,000,
wherein said protein having a molecular weight of less than 40,000 is casein.

**2.** The fluorescent nanoparticle diluent according to claim 1, wherein said protein having a molecular weight of 40,000 or higher is BSA.

**3.** The fluorescent nanoparticle diluent according to claim 1 or 2, wherein the ratio of κ-casein in said casein is 0 to 10% (W/W).

**4.** The fluorescent nanoparticle diluent according to any one of claims 1 to 3, wherein the ratio of α-casein and β-casein (α-casein:β-casein) contained in said casein is 40:60 to 60:40 (taking the total amount of α-casein and β-casein as 100).

**5.** An immunofluorescent staining kit, comprising:

(i) the fluorescent nanoparticle diluent according to any one of claims 1 to 4; and
(ii) a fluorescent nanoparticle-containing immunostaining reagent.

**6.** An immunofluorescent staining solution, comprising:

the fluorescent nanoparticle diluent according to any one of claims 1 to 4; and
fluorescent nanoparticles.

**7.** An immunofluorescent staining method, comprising the immunostaining step using the immunofluorescent staining solution according to claim 6.

**8.** The immunofluorescent staining method according to claim 7, wherein said immunostaining step comprises the step of sequentially performing:

a primary reaction treatment of specifically binding a primary antibody to a biological substance of interest;
a secondary reaction treatment of specifically binding a biotin-bound secondary antibody to said primary antibody; and
a treatment of diluting avidin-bound fluorescent nanoparticles with the fluorescent nanoparticle diluent according to any one of claims 1 to 4 and then fluorescently labeling said secondary antibody with the resulting solution.

**9.** A fluorescent nanoparticle diluent, which is used for performing a gene staining method with fluorescent nanoparticles and comprises:

1 to 5% (W/W) of a protein having a molecular weight of 40,000
or higher; and
1 to 3% (W/W) of a protein having a molecular weight of less than 40,000,
wherein said protein having a molecular weight of less than 40,000 is casein.

**10.** The fluorescent nanoparticle diluent according to claim 9, wherein said protein having a molecular weight of 40,000 or higher is BSA.

**11.** The fluorescent nanoparticle diluent according to claim 9 or 10, wherein the κ-casein content in said casein is 10% (W/W) or less.

**12.** The fluorescent nanoparticle diluent according to any one of claims 9 to 11, wherein the ratio of α-casein and β-casein (α-casein:β-casein) contained in said casein is 40:60 to 60:40 (taking the total amount of α-casein and β-casein as 100).

**13.** A gene staining kit, comprising:

(i) the fluorescent nanoparticle diluent according to any one of claims 9 to 12; and
(ii) a fluorescent nanoparticle-containing gene staining reagent.

**14.** A gene staining solution, comprising:

the fluorescent nanoparticle diluent according to any one of claims 9 to 12;

a probe; and
fluorescent nanoparticles that are capable of binding to or bound with said probe.

15. A gene staining method, comprising the gene staining step using the gene staining solution according to claim 14.

16. The gene staining method according to claim 15, wherein said gene staining step comprises the step of sequentially performing:

a primary reaction treatment of specifically binding a first binding group molecule-containing probe to a gene to be detected; and
a secondary reaction treatment of diluting fluorescent nanoparticles bound with a second binding group molecule, which specifically binds to said first binding group molecule, with the fluorescent nanoparticle diluent according to any one of claims 9 to 12 and then fluorescently labeling said probe specifically bound to said gene to be detected with the resulting solution.

Europäisches
Patentamt

European
Patent Office

Office européen
des brevets

# EUROPEAN SEARCH REPORT

Application Number

EP 20 16 4877

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/224770 A1 (KVAM ERIK LEEMING [US] ET AL) 29 August 2013 (2013-08-29) * see [0032], example 5 * | 1-16 | INV. G01N33/543 G01N33/531 G01N33/533 G01N21/64 G01N33/574 G01N33/58 |
| A | EP 1 508 805 A1 (SEIKAGAKU KOGYO CO LTD [JP]) 23 February 2005 (2005-02-23) * [0009-0029, 0049-0060, 0067-0073], Tables 1-2, claims * | 1-16 | |
| X | JP 2012 194013 A (KONICA MINOLTA MEDICAL & GRAPHIC INC; UNIV TOHOKU) 11 October 2012 (2012-10-11) | 1,3-9, 11-16 | ADD. G01N33/536 G01N33/53 G01N33/48 |
| A | * abstract * | 2,10 | |
| X | US 2005/214882 A1 (ZOU RAN [US] ET AL) 29 September 2005 (2005-09-29) | 1,3-9, 11-16 | |
| A | * [0059-0062, 0072], examples 3-4 * | 2,10 | |
| A | EP 2 613 145 A1 (KONICA MINOLTA MED & GRAPHIC [JP]; UNIV TOHOKU [JP]) 10 July 2013 (2013-07-10) * [0016-0019, 0037-0045, 0050, 0055-0057] * | 1-16 | |
| A | EP 2 541 248 A2 (KOREA FOOD RES INST [KR]) 2 January 2013 (2013-01-02) * [0023, 0034-0039], Fig. 3 * | 1-16 | TECHNICAL FIELDS SEARCHED (IPC) G01N |
| A | A. A. Y. GIBRIEL: "Options available for labelling nucleic acid samples in DNA microarray-based detection methods", BRIEFINGS IN FUNCTIONAL GENOMICS, vol. 11, no. 4, 17 April 2012 (2012-04-17), pages 311-318, XP055325116, Oxford, UK ISSN: 2041-2649, DOI: 10.1093/bfgp/els015 * page 315 - page 316 * | 1-16 | |

The present search report has been drawn up for all claims

| Place of search | Date of completion of the search | Examiner |
|---|---|---|
| The Hague | 14 April 2020 | Diez Schlereth, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 4877

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2020

| Patent document cited in search report | | Publication date | Patent family member(s) | | Publication date |
|---|---|---|---|---|---|
| US 2013224770 | A1 | 29-08-2013 | EP | 2820421 A1 | 07-01-2015 |
| | | | US | 2013224770 A1 | 29-08-2013 |
| | | | WO | 2013130002 A1 | 06-09-2013 |
| EP 1508805 | A1 | 23-02-2005 | CA | 2477991 A1 | 20-02-2005 |
| | | | EP | 1508805 A1 | 23-02-2005 |
| | | | EP | 2031390 A1 | 04-03-2009 |
| | | | US | 2005100935 A1 | 12-05-2005 |
| | | | US | 2013273048 A1 | 17-10-2013 |
| JP 2012194013 | A | 11-10-2012 | NONE | | |
| US 2005214882 | A1 | 29-09-2005 | CN | 101421619 A | 29-04-2009 |
| | | | US | 2005214882 A1 | 29-09-2005 |
| | | | US | 2010041076 A1 | 18-02-2010 |
| | | | US | 2011244494 A1 | 06-10-2011 |
| | | | WO | 2005094539 A2 | 13-10-2005 |
| EP 2613145 | A1 | 10-07-2013 | EP | 2613145 A1 | 10-07-2013 |
| | | | EP | 3225990 A1 | 04-10-2017 |
| | | | JP | 5915531 B2 | 11-05-2016 |
| | | | JP | 5924396 B2 | 25-05-2016 |
| | | | JP | 6112173 B2 | 12-04-2017 |
| | | | JP | 6128153 B2 | 17-05-2017 |
| | | | JP | 2015062032 A | 02-04-2015 |
| | | | JP | 2015062033 A | 02-04-2015 |
| | | | JP | 2015111167 A | 18-06-2015 |
| | | | JP | 2016029387 A | 03-03-2016 |
| | | | JP | WO2012029342 A1 | 28-10-2013 |
| | | | US | 2013157895 A1 | 20-06-2013 |
| | | | US | 2015160103 A1 | 11-06-2015 |
| | | | WO | 2012029342 A1 | 08-03-2012 |
| EP 2541248 | A2 | 02-01-2013 | CN | 102859359 A | 02-01-2013 |
| | | | CN | 104316678 A | 28-01-2015 |
| | | | CN | 104316697 A | 28-01-2015 |
| | | | CN | 104316698 A | 28-01-2015 |
| | | | EP | 2541248 A2 | 02-01-2013 |
| | | | KR | 20110096739 A | 31-08-2011 |
| | | | US | 2013029428 A1 | 31-01-2013 |
| | | | WO | 2011105721 A2 | 01-09-2011 |

EPO FORM P0459

For more details about this annex : see Official Journal of the European Patent Office, No. 12/82

**EP 3 690 443 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013057631 A
- JP 2006053031 A

- JP 2013057937 A

**Non-patent literature cited in the description**

- *Cell Biochem. Biophys.,* 2006, vol. 45 (1), 59